# EUROPEAN PATENT APPLICATION

(11) **EP 4 659 758 A1**
(43) Date of publication of application: **10.12.2025**
(21) Application number: 24750273.5
(22) Date of filing: 30.01.2024
(51) Int. Cl.: A61K 38/06, A61K 8/64, A61P 17/00, A61P 17/16, A61P 43/00, A61Q 19/00, A61Q 19/02, A61Q 19/08

(54) **ANTI-AGING COMPOSITION**

(30) Priority: 31.01.2023 JP 2023013251; 20.06.2023 JP 2023100465
(71) Applicant: Mitsubishi Corporation Life Sciences Limited, Tokyo 100-0006 (JP)
(72) Inventor: UCHIDA Yoshiaki, Tokyo 100-0006 (JP); ITO Masayuki, Tokyo 100-0005 (JP)
(74) Representative: Müller-Boré & Partner Patentanwälte PartG mbB
(86) International application number: PCT/JP2024/002804
(87) International publication number: WO 2024/162324

(57) **Abstract**

[Problem] An object of the present invention is to provide an external composition for anti-aging, and to provide an aging retarding method using the composition.

[Solution] By using an external composition for anti-aging containing glutathione persulfide or glutathione analog persulfide, it is possible to suppress a decrease in cell proliferation capability and hyaluronic acid production capability after aging induction. In addition, it is possible to suppress tyrosinase activity of melanin induced melanoma cells, and it is possible to improve dark spots, freckles, pigmentation after sunburn, and the like by suppressing melanin production.

## Description

### Technical Field

The present invention relates to an anti-aging composition and an aging retarding method.

### Background Art

It is known that exposure to ultraviolet rays causes photoaging, inflammation, pigmentation, and the like. In particular, it is known that aging progresses due to genetic factors and environmental factors, which causes a decrease in the number of skin cells and a decrease in skin function such as hyaluronic acid production, and causes a decrease in skin freshness and firmness, stains, and wrinkles. In addition, it is known that dark spots, freckles, pigmentation after sunburn, and the like are caused by significantly enhanced melanin production in pigmented cells present in the skin.

Hitherto, as a method for retarding aging of cells, there have been reported a fibroblast activator containing niacinamide, a hydrolyzate of a rice bran extract, and an extract of bamboo shoot as active ingredients (Patent Literature 1), and a composition for preventing or treating photoaging, inflammation, pigmentation, and/or cancer by ultraviolet rays (Patent Literature 2), which contains a silk tree extract. However, since the silk tree and the bamboo shoot are natural products, there has been a problem that the components contained therein vary depending on the period.

In addition, as a method for suppressing melanin production, a melanin production suppressing agent containing a yeast extract and glutathione and a cosmetic containing the melanin production suppressing agent have been reported (Patent Literature 3). In addition, as a sulfur-containing cosmetic, a combination of levulinic acid and a sulfur compound has been reported (Patent Literature 4). However, compounds such as reduced glutathione and a sulfide salt have a sulfur-like odor, and oxidized glutathione has a problem in terms of titer. For this reason, a composition having a high titer, less odor, and a melanin production suppressing action has been required.

In addition, a method for retarding aging by promoting glutathione production in vivo by administering a polyamine has been reported (Patent Literature 5). In recent years, persulfides have attracted attention as glutathione-related substances, and their antioxidant activity has been expected; however, aging retarding using persulfides has not been reported so far.

### Citation List

### Patent Literature

Patent Literature 1: JP 2022-138725 A
Patent Literature 2: JP 2022-149769 A
Patent Literature 3: WO 2015/151867 A
Patent Literature 4: JP 2002-326918 A
Patent Literature 5: JP 2021-050180 A

### Summary of Invention

### Technical Problem

An object of the present invention is to provide an anti-aging composition containing glutathione persulfide or glutathione analog persulfide, and to provide a method for retarding aging by an anti-aging external composition containing glutathione persulfide or glutathione analog persulfide.

### Solution to Problem

As a result of intensive studies on the solution of the above problems, the present inventors have found that glutathione persulfide suppresses aging of cells subjected to aging induction treatment, and have completed the present invention.

That is, the present invention relates to the following first to sixth aspects.

First, the anti-aging composition contains 0.001 to 0.7 wt% of persulfide of a glutathione analog or persulfide of a cysteine analog.

Second, the present invention provides an anti-aging composition in which the persulfide of a glutathione analog or the persulfide of a cysteine analog is oxidized glutathione trisulfide or oxidized NAC trisulfide.

Third, the aging retarding method includes using the composition described in the first or second aspect as an oral preparation or an external preparation.

Fourth, the aging retarding method according to the third aspect, wherein the aging retarding method is a hyaluronic acid production enhancing method or a melanin production suppressing method.

Fifth, there is provided a method for enhancing a melanin production suppressing action of a glutathione analog or a cysteine analog, wherein the glutathione analog or the cysteine analog is persulfided.

Sixth, there is provided a method for enhancing a hyaluronic acid producing action of a glutathione analog or a cysteine analog, wherein the glutathione analog or the cysteine analog is persulfided.

### Advantageous Effects of Invention

According to the present invention, by using an anti-aging composition containing glutathione persulfide or glutathione analog persulfide, it is possible to suppress a decrease in cell proliferation capability and a decrease in hyaluronic acid production due to aging induction in normal human fibroblasts and normal human epidermal keratinocytes which have been induced to age, and it is possible to improve wrinkles of the skin and to improve firmness and radiance.

### Brief Description of Drawings

Fig. 1 illustrates suppression decrease in proliferation capability of aging-induced normal human fibroblasts and in proliferation capability due to persulfide.
Fig. 2 illustrates suppression decrease in proliferation capability of aging-induced normal human epidermal keratinocytes and in proliferation capability due to persulfide.
Fig. 3 illustrates suppression decrease in hyaluronic acid production capability of aging-induced normal human fibroblasts and in hyaluronic acid production capability due to persulfide.
Fig. 4 illustrates suppression decrease in hyaluronic acid production capability of aging-induced normal human epidermal keratinocytes and in hyaluronic acid production capability due to persulfide.
Fig. 5 illustrates results of a melanin production suppression test.
Fig. 6 illustrates results of a tyrosinase activity inhibition test.
Fig. 7 illustrates a change in ΔL value of human skin due to persulfide.
Fig. 8 illustrates a change in Δa value of human skin due to persulfide.
Fig. 9 illustrates a change in Δb value of human skin due to persulfide.

### Description of Embodiments

The composition of the invention includes glutathione analog persulfide or cysteine analog persulfide (Hereinafter, it may be collectively referred to as persulfide.). Here, the glutathione analog in the present invention refers to reduced or oxidized glutathione, reduced or oxidized γ-glutamylcysteine, reduced or oxidized cysteinylglycine, those in which one or more amino groups or carboxyl groups in these structures are modified with an alkyl group having a linear or branched structure with 1 to 24 carbon atoms, and esterified with an organic acid or an inorganic acid. In addition, the cysteine analog in the present invention refers to cysteine, cystine, reduced or oxidized N-acetylcysteine (Hereinafter, it may be referred to as NAC.), one in which one or more amino groups or carboxyl groups in these structures are modified with an alkyl group having a linear or branched structure having 1 to 24 carbon atoms, and esterified with an organic acid or an inorganic acid. The persulfide in the present invention is a molecule in which one or more sulfur atoms are further directly bonded to a thiol group or a sulfur atom constituting a disulfide bond present in a molecule of an ordinary glutathione analog or cysteine analog (for example, R-S-SH or R-S-S-S-R'; R and R' refer to any functional groups). One in which one or more sulfur atoms are further directly bonded to a sulfur atom in the molecule of a glutathione analog or a cysteine analog can be used in the present invention, and may be used as a mixture in which a plurality of compounds having different numbers of sulfur atoms in the molecule are present.

The persulfide contained in the composition of the present invention is preferably an oxidized type, more preferably oxidized glutathione persulfide or oxidized NAC persulfide, and still more preferably oxidized glutathione persulfide (oxidized glutathione trisulfide) or oxidized NAC persulfide (oxidized NAC trisulfide) in which three sulfur atoms are included in one molecule of the persulfide.

In the method for obtaining the persulfide of the present invention, the persulfide can be produced using the glutathione analog or cysteine analog which is generally available as a raw material. For example, oxidized glutathione and peracetic acid are reacted on ice for 30 minutes, a solvent obtained by mixing equal amounts of ethanol and THF (tetrahydrofuran) is added, and then a precipitate obtained by centrifugation is further reacted with sodium hydrogen sulfide at room temperature, whereby oxidized glutathione persulfide can be obtained. By purifying this oxidized glutathione persulfide, oxidized glutathione trisulfide can be produced, and it is possible to obtain crystalline, amorphous, solution, suspension, and other forms.

The composition of the present invention contains the persulfide as an active ingredient, and may be used as a mixture with other materials as long as the effect of the present invention is not impaired. In addition to the above-mentioned persulfide, thickeners, pH adjusting agents, perfumes, bases for external preparations, and the like can be mentioned.

The persulfide may be prepared by mixing the persulfide with other raw materials at the same time when preparing the composition, or may be prepared by mixing the persulfide with other raw materials at a different timing. The form of the composition is not limited as long as it can be orally ingested or applied to the skin, and it can be prepared and used in the form of a solution, a dispersion liquid, a powder, a granule, a capsule, a tablet, a paste, a gel, or a sheet according to a conventional method using a solvent or a base material used for general foods, supplements, cosmetic compositions, pharmaceutical compositions, and external preparations for skin.

The content of each component in the composition of the present invention can be appropriately adjusted by adding an excipient or the like generally used as an oral composition or an external preparation for skin.

The content of the persulfide contained in the composition of the present invention can be known by a general method using HPLC. The content of the persulfide in the composition of the present invention may be appropriately adjusted, and the composition usually contains 0.001 to 0.7 wt%, preferably 0.0012 to 0.5 wt% of the persulfide.

The aging in the present invention refers to an increase in wrinkles of the skin, a decrease in firmness and radiance, a decrease in the moisture-retaining capacity of the skin, occurrence of dark spots, and pigmentation, and the anti-aging action of the present invention refers to an improvement in wrinkles of the skin, an improvement in firmness, radiance, or brightness, an improvement in the moisture-retaining capacity of the skin (moisturization), and suppression of pigmentation. It is considered that the hyaluronic acid production amount is related to an increase in wrinkles of the skin, a decrease in firmness and radiance, and a decrease in the moisture-retaining capacity of the skin. In addition, it is considered that melanin production is involved in the occurrence of dark spots and pigmentation. The degree of aging can be determined from, for example, any commonly used aging index such as a decrease in cell proliferation capability and hyaluronic acid production amount and melanin production amount in cultured cells, such as an increase in wrinkles, a decrease in firmness and radiance, a decrease in a moisture-retaining capacity of skin, or occurrence of dark spots and pigmentation in human skin.

In addition, it is possible to know the anti-aging effect (aging retarding effect) of the anti-aging composition by using, as an index, how much the aging index such as the decrease in cell proliferation capability, hyaluronic acid production capability, or melanin production amount can be improved.

For example, since it is known that cell proliferation capability is reduced in aging cells, evaluation can be performed by a general cell proliferation capability test system such as an MTT assay. In addition, the degree of suppression of the decrease in hyaluronic acid production amount or the degree of suppression of melanin production can be evaluated. The hyaluronic acid production capability in aging cells can be evaluated by quantifying it by a general method such as ELISA.

In addition, the composition of the present invention exhibits a tyrosinase activity inhibitory action, and can suppress melanin production to suppress the occurrence of dark spots and pigmentation. The amount of melanin in the cultured cells can be quantified by a general method, and examples thereof include a method of measuring an absorbance at 405 nm. In addition, in a case where the melanin amount of the skin is measured, for example, the amount of melanin can be quantified by measurement using Mexameter. The melanin production suppressing effect can be known by the decrease in the amount of melanin measured by any of the methods.

In addition, the tyrosinase activity in the present invention can be confirmed by adding L-DOPA having a known concentration to a protein extract containing tyrosinase derived from B16 mouse melanoma cells in which melanin is induced by a melanin inducer such as α-MSH (melanocyte stimulating hormone) to react, measuring the absorbance at 490 nm, and correcting the absorbance with the protein concentration in the protein extract. The tyrosinase activity inhibitory effect can be known by comparing the value of the absorbance at 490 nm corrected by the protein concentration measured by adding the test substance at the time of melanin induction and the same operation with the value of the test section to which the test substance is not added.

Since the melanin production suppressing composition contains persulfide as an active ingredient, the composition can be used as a composition having a melanin production suppressing action by being directly applied to the skin as an external preparation or orally ingested.

In addition, as described above, a desired dosage form may be prepared and used.

Since the anti-aging composition contains persulfide as an active ingredient, the composition can be used as a composition having an anti-aging action by being applied to the skin as it is as an external preparation.

In addition, as described above, a desired dosage form may be prepared and used.

### Examples

Hereinafter, the present invention will be specifically described with reference to Examples, but the present invention is not limited thereto.

The persulfide was produced by the following method. Oxidized glutathione (NACALAI TESQUE, INC.) was dissolved in ultrapure water to prepare a 170 mM (mol/L) oxidized glutathione aqueous solution. To 2.5 mL of this oxidized glutathione aqueous solution, 4 mL of a 32 wt% peracetic acid solution (Sigma-Aldrich) was added, and the mixture was reacted on ice for 30 minutes. 30 mL of a solvent obtained by mixing equal amounts of ethanol and THF (both available from NACALAI TESQUE, INC.) was added to the obtained reaction solution, and the mixture was centrifuged at 10,000 rpm for 10 minutes at 4°C, and the recovered precipitate was dried under reduced pressure for 1 hour. The obtained dried product was dissolved by adding 2.5 mL of ultrapure water. An equal amount of sodium hydrogen sulfide solution prepared to 60 mM with 0.3M NaOH was further added, and the mixture was reacted at room temperature for 1 hour to obtain oxidized glutathione persulfide. The obtained oxidized glutathione persulfide was purified using a synthetic adsorption resin, concentrated, and then dried to obtain a powder of oxidized glutathione trisulfide (Sample 1).

In addition, the same procedure was carried out except that N-acetylcysteine (NACALAI TESQUE, INC.) was used instead of oxidized glutathione to obtain oxidized NAC trisulfide powder (sample 2).

### (Suppression of decrease in proliferation capability of normal human fibroblast)

Normal human fibroblasts were seeded in 96 well plates (3.0 × 10^3 cells/well, N = 3) and precultured for 24 hours (5% CO2, 37°C). As a medium, MEM-α, nucleosides medium (Gibco) containing fetal bovine serum (10% of serum-free medium was externally added) was used. Thereafter, the wells were washed once with PBS (-), then replaced with MEM-α, nucleoside medium containing fetal bovine serum (5% of serum-free medium was externally added) dissolved so that the final concentration of Sample 1 or Sample 2 was 50 µM or 100 µM, and further incubated for 30 minutes. Thereafter, fibroblasts were treated with a medium adjusted so that the final concentration of hydrogen peroxide was 0.5 mM to induce aging. Thereafter, an MTT assay was performed according to a conventional method, and the survival rate of fibroblasts was calculated as a relative value when the survival rate of cells in the test section in which neither treatment nor aging induction was performed was taken as 100.

For comparison, a test section in which only aging induction was performed and a test section in which reduced glutathione was 100 µM was set instead of Sample 1.

With respect to the survival rate, a significant difference test (t-test, two-tailed) between the two groups of the test section of Sample 1 and the test section of reduced glutathione and the test section of Sample 2 and the test section of reduced glutathione was performed respectively.

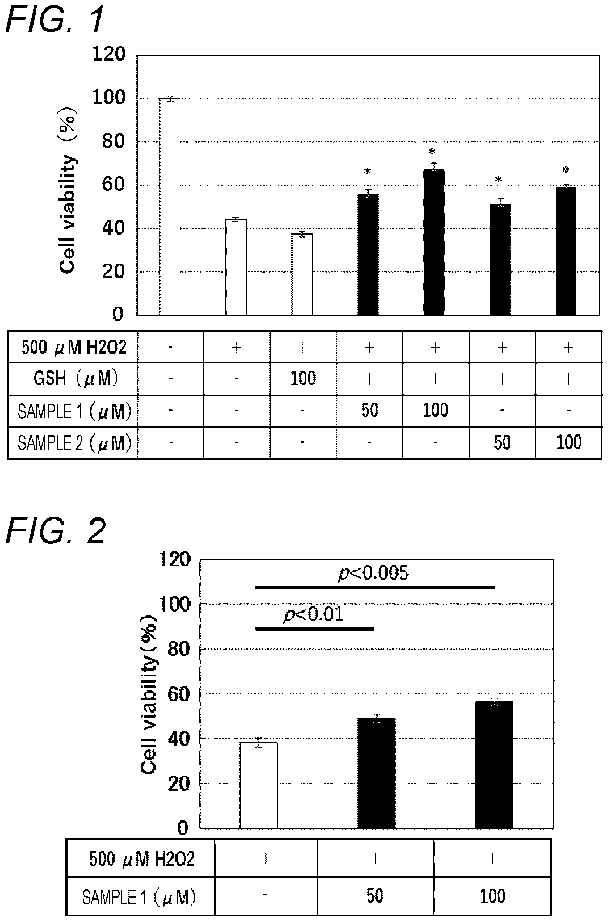

The results are illustrated in Fig. 1. The test section of Sample 1 and Sample 2 showed a significant cell proliferation rate (both p < 0.005) as compared with the test section of only aging induction and the test section of reduced glutathione. That is, it was revealed that oxidized glutathione trisulfide and oxidized NAC trisulfide can significantly suppress a decrease in cell proliferation capability due to aging induction of normal human fibroblasts.

### (Suppression of decrease in proliferation capability of normal human epidermal keratinocytes)

Normal human epidermal keratinocytes were seeded in 96 well plates (1.0 × 10^4 cells/well, N = 3) and precultured for 24 hours (5%CO2, 37°C). As a medium, a commercially available keratinocyte growth medium (PromoCell) was used. Thereafter, the wells were washed once with PBS (-), then replaced with a keratinocyte growth medium dissolved so that the final concentration of Sample 1 was 50 µM or 100 µM, and further incubated for 30 minutes. Thereafter, normal human epidermal keratinocytes were treated with a medium adjusted to have a final concentration of hydrogen peroxide of 0.5 mM to induce aging. Thereafter, an MTT assay was performed according to a conventional method, and the survival rate of normal human epidermal keratinocytes was calculated as a relative value when the survival rate of cells in a test section in which neither the treatment with oxidized glutathione trisulfide nor the aging induction was performed was taken as 100.

For comparison, a test section in which only aging induction was performed without treatment with oxidized glutathione trisulfide was set.

Regarding the survival rate, a significant difference test (t-test, two-tailed) between the two groups of the test section of Sample 1 and the test section in which only aging induction was performed was performed respectively.

The results are illustrated in Fig. 2. All of the test sections of Sample 1 showed a significant cell proliferation rate as compared with the test section with only aging induction. That is, it was revealed that oxidized glutathione trisulfide can significantly suppress a decrease in cell proliferation capability due to aging induction of normal human epidermal keratinocytes.

### (Suppression of decrease in hyaluronic acid production capability due to aging induction)

Normal human fibroblasts were seeded in 24 well plates (8.0 × 10^4 cells/well, N = 3) and precultured for 24 hours (5% CO2, 37°C). As a medium, MEM-α, nucleosides medium (Gibco) containing fetal bovine serum (10% of serum-free medium was externally added) was used. Thereafter, the wells were washed once with PBS (-), then replaced with MEM-α, nucleoside medium containing fetal bovine serum (5% of serum-free medium was externally added) dissolved so that the final concentration of Sample 1 or Sample 2 was 50 µM or 100 µM, and further incubated for 30 minutes. Thereafter, fibroblasts were treated with a medium adjusted so that the final concentration of hydrogen peroxide was 0.5 mM to induce aging. Thereafter, the culture medium was changed to MEM-α, nucleoside medium containing fetal bovine serum (5% of serum-free culture medium was externally added) and incubated for 24 hours, and then the amount of hyaluronic acid was quantified by ELISA using a hyaluronic acid quantification kit (Cosmo Bio Inc.).

The hyaluronic acid production amount in each test section was shown as a relative value when the hyaluronic acid production amount in each sample and the test section in which aging induction was not performed was taken as 100. Regarding the hyaluronic acid production amount, a significant difference test (t-test, two-tailed) between the two groups of the test section for Sample 1 and the test section for which only the aging induction was performed respectively, and a significant difference test between the test section for Sample 2 and the test section for which only the aging induction was performed were each performed, and "*(p < 0.05)" was marked for those in which a significant difference was recognized.

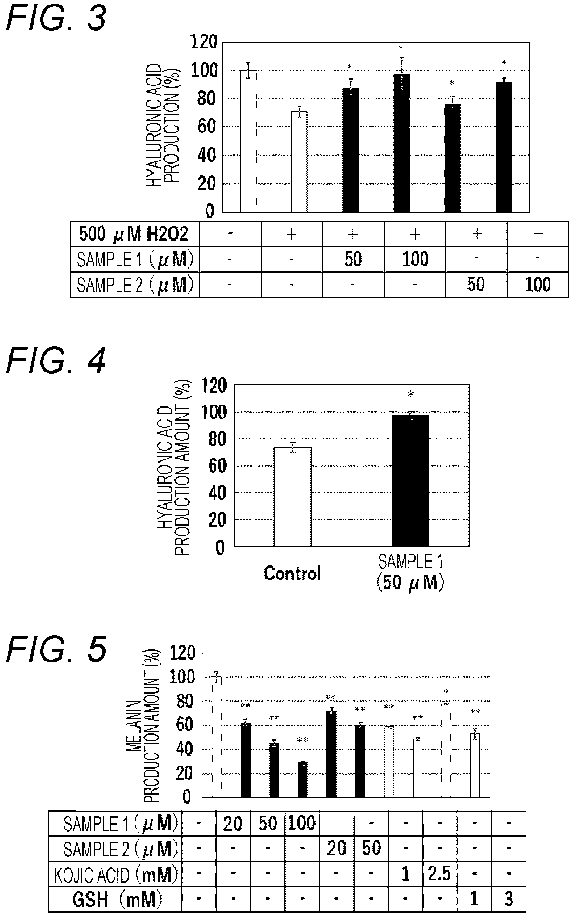

The results are illustrated in Fig. 3. It was revealed that the amount of hyaluronic acid in the test section of Sample 1 or Sample 2 was significantly higher than that in the test section of only aging induction. In addition, all of the test sections of Sample 1 showed the same hyaluronic acid production capability as those of the test sections that did not induce aging, and no significant difference was recognized between the two groups. The same result was obtained for the test section of Sample 2. Therefore, it was suggested that oxidized glutathione trisulfide and oxidized NAC trisulfide can suppress a decrease in hyaluronic acid production capability due to aging induction of normal human fibroblasts.

### (Suppression of decrease in hyaluronic acid production capability due to aging induction)

Normal human epidermal keratinocytes were seeded in 24 well plates (1.0 × 10^5 cells/well, N = 3) and precultured for 24 hours (5%CO2, 37°C). As a medium, a commercially available keratinocyte growth medium (PromoCell) was used. Thereafter, the wells were washed once with PBS (-), then replaced with a keratinocyte growth medium dissolved so that the final concentration of Sample 1 was 50 µM, and further incubated for 30 minutes. Thereafter, normal human epidermal keratinocytes were treated with a medium adjusted to have a final concentration of hydrogen peroxide of 0.5 mM to induce aging. Thereafter, the medium was replaced with a new keratinocyte growth medium and incubated for 24 hours, and then the amount of hyaluronic acid was quantified by an ELISA method using a hyaluronic acid quantification kit (Cosmo Bio). For comparison, a test section not treated with oxidized glutathione trisulfide was provided.

The hyaluronic acid production amount in each of the test sections was shown as a relative value when the hyaluronic acid production amount in the test section in which neither oxidized glutathione trisulfide treatment nor aging induction was performed was taken as 100. Regarding the hyaluronic acid production amount, a significant difference test (t-test, two-tailed) between the two groups of the test section of Sample 1 and the test section of only aging induction was performed, and "*(p < 0.05)" was marked for those in which a significant difference was recognized.

The results are illustrated in Fig. 4. It was revealed that the amount of hyaluronic acid in the test section of Sample 1 was significantly higher than that in the test section of only aging induction. Therefore, it was suggested that aging was retarded.

In addition, the test section of Sample 1 showed the same hyaluronic acid production capability as that of the control group in which aging is not induced, and no significant difference was recognized between these two groups. Therefore, it was suggested that oxidized glutathione trisulfide can suppress a decrease in hyaluronic acid production capability due to aging induction of normal human epidermal keratinocytes. From previous tests, it is presumed that oxidized NAC trisulfide has a similar effect.

### <Melanin production suppression test in cultured cells>

B16 mouse melanoma cells were seeded in a 6 well plate (2.0 × 10^4 cells/well), and cultured for 24 hours in D-MEM medium to which 5% fetal bovine serum was added with respect to the medium (5%CO2, 37°C). Thereafter, α-MSH (final concentration: 200 nM) was added to a serum-free D-MEM medium and cultured for 24 hours to induce melanin. After melanin induction, the cells were washed with PBS (-), then treated with 0.25% trypsin (containing EDTA) and recovered, and centrifuged at 10,000 rpm for 3 minutes to obtain pellets. The obtained pellets were washed twice with PBS (-), 150 µL of a 1 M sodium hydroxide solution was then added, and the mixture was treated at 100°C for 10 minutes to dissolve the pellets, thereby preparing a reference sample.

In addition, a test section of Sample 1 (final concentration: 0.02 mM or 0.05 mM) or Sample 2 (final concentration: 0.02 mM or 0.05 mM) at the time of melanin induction, and kojic acid (final concentration: 1 mM or 2.5 mM) or reduced glutathione (final concentration: 1 mM or 3 mM) as a comparison target was set, and each sample was prepared.

One hundred µL of a sample of each test section was added to a 96 well plate, and the absorbance at 405 nm was measured with a microplate reader (n = 3). The relative value of each test section was calculated when the measured value of the reference sample was 100. The significant difference test was performed on the measured value of the reference sample by the Dunnett method (*p < 0.05, **p *<* 0.01.).

The results are illustrated in Fig. 5. When compared with the measured value of the reference sample, it was revealed that the melanin production amount was significantly low in both of the test sections of Sample 1 and Sample 2 (*p* < 0.01). In addition, the 0.02 mM test section of Sample 1 and the test section to which kojic acid, which is known to suppress melanin production, was added had a similar melanin production suppressing effect, but it was revealed that the concentration of oxidized glutathione trisulfide was 0.02 mM, whereas the kojic acid to be compared was 1 mM, and oxidized glutathione trisulfide exhibited a melanin production suppressing effect at a lower concentration. Furthermore, the test section (final concentration: 3 mM) to which reduced glutathione was added also showed the same melanin production suppressing effect as the 0.02 mM test section of Sample 1, but oxidized glutathione trisulfide showed the melanin production suppressing effect at a lower concentration. Also in the test section of Sample 2, it was shown that melanin production can be suppressed at a lower concentration as compared with the compound to be compared. From the above, oxidized glutathione trisulfide has an excellent melanin production suppressing effect on melanin production in melanoma cells as compared with reduced glutathione or kojic acid.

### <Tyrosinase activity suppression test>

B16 mouse melanoma cells were seeded in a 6 well plate (2.0 × 10^4 cells/well), and cultured for 24 hours in D-MEM medium to which 5% fetal bovine serum was added with respect to the medium (5%CO2, 37°C). Thereafter, α-MSH (final concentration: 200 nM) was added, and the cells were cultured in a serum-free D-MEM medium for 24 hours to induce melanin. After induction of melanin, the cells were washed twice with PBS (-), recovered by treatment with 0.25% trypsin (containing EDTA), and centrifuged at 10,000 rpm for 3 minutes to obtain pellets. The obtained pellets were washed twice with PBS (-), and then solubilized in 100 µL of a solubilization buffer (RIPA Buffer; Fujifilm Wako Pure Chemical Industries, Ltd.) and centrifuged (14,000 rpm, 15 min) at 4°C, and the supernatant was used as a sample of Comparative Example 5. The protein concentration of the obtained sample was measured by the BCA method.

The same operation was performed except that melanin induction was not performed, and a reference sample was obtained. In addition, a test section of Sample 1 (final concentration: 0.05 mM or 0.1 mM) at the time of melanin induction, and a test section in which only melanin induction was performed as a comparison target were provided. Fifty µL of a sample of each test section was placed in a 96 well plate (n=3), 50 µL of 0.8 mg/mL L-DOPA was added, the mixture was reacted at 37°C for 1 hour, and then the absorbance at 490 nm was measured with a microplate reader. The absorbance was corrected by the protein concentration, and the relative value of each test section was calculated when the measured value of the reference sample was 100. The significant difference test was performed using Dunnett method for the test section in which only melanin induction was performed (*p < 0.05, ***p* < 0.01.).

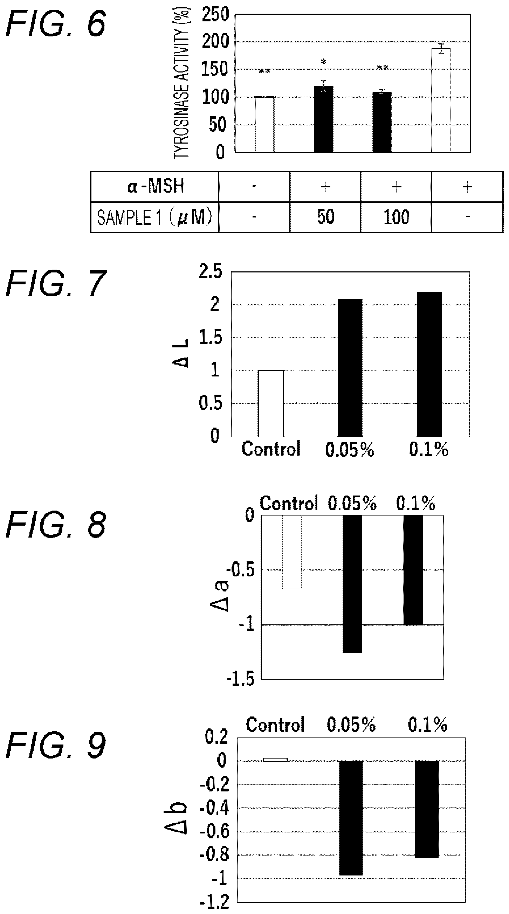

The results are illustrated in Fig. 6. The test section to which Sample 1 was added had the same degree of tyrosinase activity as the reference sample at any concentration. In addition, it was shown that the tyrosinase activity was significantly lower than the test section in which only melanin induction was performed. These results revealed that oxidized glutathione trisulfide can suppress tyrosinase activity in melanoma cells.

From the above, it was shown that the glutathione analog persulfide can inhibit tyrosinase activity in cultured cells in which melanin induction has been performed, and can suppress the melanin production amount. In addition, from the results of the test of melanin production suppression, it is presumed that oxidized NAC trisulfide exhibits a similar effect.

### <Skin whitening effect test on human>

In order to confirm the whitening effect on humans, test toners (Lotion 1 and Lotion 2) containing the composition of the present invention were prepared according to the composition in Table 1. Three subjects who agreed with the test were subjected to the test, and applied Lotion 1 and Lotion 2 to the test sites (five sites each) provided on the inner side of the right forearm and the inner side of the left forearm, respectively, twice in the morning and night (after bathing in the evening) during the period from November 10, 2023 to December 15 of the same year. The skin was measured by the following method before the start and after the end of the application period.

First, the test sites were washed with soap, dried with paper towels, and then acclimatized for 15 minutes. Next, The L* value, a* value, and b* value at five test sites were measured using a color difference meter (SE-7700; NIPPON DENSHOKU INDUSTRIES CO., LTD.) + optical fiber, and the average value was calculated. From the obtained average values of the L* value, the a* value, and the b* value before application, the difference between the average values after application was determined as a ΔL value, a Δa value, and a Δb value. A non-applied site was defined as Control.

**[Table 1]**

| Component | Lotion 1 | Lotion 2 |
|---|---|---|
| Sample 1 | 0.1 | 0.05 |
| PEG-60 hydrogenated castor oil | 0.1 | 0.1 |
| Xanthan gum | 0.1 | 0.1 |
| Butylene glycol | 1 | 1 |
| 10 mM citric acid buffer (pH 5.5) + 0.2% phenoxyethanol | Up to 100% | Up to 100% |

The results are illustrated in Figs. 7 to 9. The ΔL value tended to be larger at the sites to which Lotion 1 or 2 was applied than in the non-applied area (Control), suggesting that the lightness of the skin was improved. This result was presumed to be attributed to the melanin production suppressing effect.

In addition, the a* value is an item related to the redness of the skin, and the smaller the Δa value, the more the redness is improved. From Fig. 8, at the sites to which Lotion 1 or 2 was applied, the Δa value was smaller than that in Control. This is considered to be because the hyaluronic acid production capability was improved, so that the skin was well-conditioned and the redness was reduced.

Finally, the b* value is an item related to skin dullness, and a smaller Δb value indicates that skin dullness is improved. From Fig. 9, it was suggested that the parts to which Lotion 1 or 2 was applied had a smaller Δb value than Control, and skin dullness was improved.

Summarizing the above, it was shown that by applying Lotion 1 or 2 to the skin, the lightness of the skin is improved and the balance of the skin color is improved.

Based on the results of the cell test, it is presumed that oxidized NAC trisulfide exhibits a similar effect.

From the above, it was shown that the composition of the present invention can retard aging.

## Claims

1. An anti-aging composition comprising 0.001 to 0.7 wt% of persulfide of a glutathione analog or persulfide of a cysteine analog.

2. An anti-aging composition wherein the persulfide of a glutathione analog or the persulfide of a cysteine analog is oxidized glutathione trisulfide or oxidized NAC trisulfide.

3. An aging retarding method comprising using the composition according to claim 1 or 2 as an oral preparation or an external preparation.

4. The aging retarding method according to claim 3, wherein the aging retarding method is a hyaluronic acid production enhancing method or a melanin production suppressing method.

5. A method for enhancing a melanin production suppressing action of a glutathione analog or a cysteine analog, wherein the glutathione analog or the cysteine analog is persulfided.

6. A method for enhancing a hyaluronic acid producing action of a glutathione analog or a cysteine analog, wherein the glutathione analog or the cysteine analog is persulfided.
